Europäisches Patentamt

European Patent Office

Office européen des brevets

(19)

(11) Numéro de publication : **0 643 968 A1**

(12)

# DEMANDE DE BREVET EUROPEEN

(21) Numéro de dépôt : **94402027.0**

(51) Int. Cl.⁶ : **A61K 31/60, A61K 9/20**

(22) Date de dépôt : **12.09.94**

(30) Priorité : **14.09.93 FR 9310944**

(43) Date de publication de la demande :
**22.03.95 Bulletin 95/12**

(84) Etats contractants désignés :
**AT BE CH DE DK ES FR GB GR IE IT LI LU MC NL PT SE**

(71) Demandeur : **LABORATOIRE L. LAFON**
**19 Avenue du Professeur Cadiot**
**F-94701 Maisons Alfort (FR)**

(72) Inventeur : **Nguyen, Thanh Tam**
**27 Avenue d'alsace Lorraine**
**F-94450 Limeil Brevannes (FR)**
Inventeur : **Leyder, Joelle**
**7, rue du Cap**
**F-94000 Creteil (FR)**

(74) Mandataire : **Le Guen, Gérard et al**
**CABINET LAVOIX**
**2, place d'Estienne d'Orves**
**F-75441 Paris Cédex 09 (FR)**

(54) **Forme pharmaceutique moulée pour administration orale à base d'acide acétylsalicylique ou de ses sels et son procédé de fabrication.**

(57) La présente invention concerne une forme pharmaceutique moulée pour administration orale, stable, à base d'acide acétyl salicylique, caractérisée en ce qu'elle comprend un mélange d'un composé a) choisi parmi l'acide acétyl salicylique et ses sels pharmaceutiquement acceptables et un citrate b) choisi parmi le citrate de magnésium neutre et le citrate de magnésium acide, ce mélange étant finement réparti dans une matrice poreuse à base d'un liant pharmaceutiquement acceptable.

EP 0 643 968 A1

La présente invention concerne une forme pharmaceutique moulée pour administration orale à base diacide acétylsalicylique ou de ses sels et son procédé de préparation.

L'acide acétylsalicylique constitue un médicament très largement utilisé en raison de ses propriétés analgésiques, anti-inflammatoires et antipyrétiques.

On a également développé des sels de l'acide acétylsalicylique pour diminuer le caractère ulcérogène de l'acide acétylsalicylique.

On a cherché à mettre au point de nouvelles formes pharmaceutiques pour l'administration par voie orale d'acide acétylsalicylique ou de ses sels et notamment des formes moulées obtenues par lyophilisation d'une suspension selon la technique décrite dans FR-A-2 036 890. Les tentatives de préparation de lyophilisats moulés effectuées même en opérant à basse température (-5° C) ont mis en évidence des taux très importants de dégradation de l'acide acétylsalicylique et dissuadaient de poursuivre dans cette voie.

Par ailleurs, dans FR-A-2 670 675, on a proposé un procédé de fabrication d'une poudre à base d'acide acétylsalicylique ou d'un de ses sels pharmaceutiquement acceptable comprenant l'addition d'un composé stabilisant choisi parmi le citrate de magnésium, un mélange d'allantoïne et de chlorhydroxy aluminium et l'hydroxyde d'aluminium. A cet effet, il était nécessaire de mélanger les constituants à une température inférieure à 5° C et en pratique on opérait à une température inférieure ou égale à 0° C. De plus, en pratique, on utilisait de fortes proportions d'agent stabilisant.

On a maintenant découvert qu'il est possible d'obtenir des formes pharmaceutiques moulées pour administration orale à base d'acide acétylsalicylique ou de ses sels et qui soient stables.

On a également découvert qu'il est possible de préparer ces formes pharmaceutiques par mélange des constituants dans un milieu aqueux à la température ambiante puis lyophilisation.

L'invention a donc pour objet une forme pharmaceutique moulée pour administration orale, stable, à base d'acide acétylsalicylique, caractérisée en ce qu'elle comprend un mélange d'un composé a) choisi parmi l'acide acétylsalicylique et ses sels pharmaceutiquement acceptables et d'un citrate b) choisi parmi le citrate de magnésium neutre et le citrate de magnésium acide, ce mélange étant finement réparti dans une matrice poreuse à base d'un liant pharmaceutiquement acceptable.

L'invention a également pour objet un procédé de préparation d'une telle forme pharmaceutique comprenant le mélange, à une température inférieure ou égale à la température ambiante, dans un milieu aqueux

- d'un composé choisi parmi l'acide acétylsalicylique et ses sels pharmaceutiquement acceptables,
- d'un citrate choisi parmi le citrate de magnésium neutre et le citrate de magnésium acide,
- d'un liant,

pour former une suspension homogène, la répartition de la suspension dans des alvéoles, puis la lyophilisation de la suspension.

On a en outre découvert que la stabilité des formes pharmaceutiques était améliorée en utilisant comme citrate de magnésium le citrate de magnésium acide et que le citrate peut être présent en des proportions molaires relativement faibles (généralement de 0,1 à 3, mais avantageusement de 0,1 à 1 par rapport à l'acide acétylsalicylique ou ses sels).

Le liant constituant la matrice des formes moulées peut être notamment un colloïde ou un polymère tels que la gomme arabique, la gomme xanthane, les alginates, les pectinates, la gélatine, la cellulose, la carboxyméthylcellulose, le dextran, le chitosan, la polyvinylpyrrolidone, les polyéthylène glycols, les polymères et copolymères acryliques.

La matrice peut en outre contenir un diluant tel que lactose, mannitol, glucose, saccharose, maltodextrine, cyclodextrine et dérivés.

La suspension que l'on soumet à la lyophilisation peut en outre contenir des agents tensioactifs tels que polysorbate, ester de sorbitan, polyéther de glycéride gras, ainsi que des édulcorants et aromatisants.

Le pH de la suspension est avantageusement inférieur ou égal à 4,5. A cet effet, on peut ajouter des tampons tels que acide citrique (formant un tampon avec le citrate de magnésium), phosphate disodique, hydroxyde de magnésium, hydroxyde d'aluminium, TRIS (trométhamine), glycocolle.

La préparation de la suspension est avantageusement effectué par mélange à sec de l'acide acétylsalicylique ou d'un de ses sels avec le citrate de magnésium et éventuellement le diluant, puis incorporation à température ambiante (18-25° C) à ce mélange d'une solution aqueuse contenant le liant et les autres additifs possibles (agent tensioactif, édulcorant et aromatisants) et homogénéisation, notamment sous pression réduite (environ 0,2 à $1,0.10^2$ Pa) afin d'éviter l'incorporation d'air dans la suspension.

La suspension obtenue est ensuite distribuée dans des alvéoles préformés puis congelée à une température de -20 à -50° C pendant au moins une heure, puis lyophilisée.

La forme pharmaceutique obtenue est poreuse et stable. Elle peut être administrée avec ou sans eau.

Les exemples suivants illustrent la présente invention.

Dans ces exemples les quantités sont données en mg.

2

Le citrate de magnésium acide utilisé est le citrate pentahydraté $C_6H_5MgO_7$, $5H_2O$.

Le citrate de magnésium neutre est le citrate $Mg_3(C_6H_5O_7)_2$, $9H_2O$.

Les suspensions ont été préparées à température ambiante par mélange de l'acide acétylsalicylique et du diluant à sec et addition d'une solution aqueuse contenant le liant, l'agent tensioactif, les édulcorants et les aromatisants. Après distribution dans des alvéoles préformés, la suspension est congelée à environ -40°/-45° C pendant une heure, puis lyophilisée.

Dans ces exemples, la stabilité de l'acide acétylsalicylique a été évaluée dans la suspension avant lyophilisation et dans la forme pharmaceutique après stockage à température ambiante (environ 25° C). Les résultats d'analyse de dosage de l'acide salicylique par HPLC (exprimé en %) sont donnés dans les tableaux qui suivent.

EXEMPLE 1 : Forme contenant 80 mg d'acide acétylsalicylique

|  | 1.1 | 1.2 | 1.3 | 1.4 | 1.5 | 1.6 |
|---|---|---|---|---|---|---|
| Acide acétylsa-licylique (ASA) | 80 | 80 | 80 | 80 | 80 | 80 |
| Citrate de ma-gnésium acide | 130 | 250 | 300 | 250 | 160 | 140 |
| Aspartam | 10 | 10 | 10 | 10 | 10 | 10 |
| Dextran 70 | 70 | 70 | 70 | 60 | 80 | 90 |
| Lactose | 480 | 360 | 310 | 370 | -- | -- |
| Mannitol | -- | -- | -- | -- | 470 | 480 |
| Eau | 380 | 380 | 380 | 380 | 380 | 380 |
| pH de la suspen-sion | 3,5 | 3,3 | 3,2 | 3,3 | 3,5 | 3,5 |
| Masse unitaire après lyophili-sation | 770 | 770 | 770 | 770 | 800 | 800 |
| % d'acide sali-cylique dans la suspension au moment de l'in-troduction ASA | 0,12 | 0,10 | 0,04 | 0,09 | 0,09 | 0,12 |
| Stabilité 1 mois dans le lyophi-lisat oral conservé à tem-pérature ambian-te 1 mois | 0,39 | 0,26 | 0,29 | 0,18 | 0,17 | 0,21 |

| Stabilité 13 mois dans le lyophilisat oral conservé à température ambiante environ 13 mois | 1,25 | 0,63 | 0,79 | 0,44 | 0,70 | 0,53 |

**Exemple 2 : Forme d'acide acétylsalicylique à 160 mg**

| | 2.1 | 2.2 | 2.3 | 2.4 | 2.5 |
|---|---|---|---|---|---|
| Acide acétylsalicylique (ASA) | 160 | 160 | 160 | 160 | 160 |
| Citrate de magnésium acide | 260 | 240 | 220 | 240 | 260 |
| Aspartam | 15 | 15 | 15 | 15 | 15 |
| Dextran 70 | 70 | 70 | 70 | 60 | 70 |
| Lactose | 315 | 335 | 355 | 345 | -- |
| Mannitol | -- | -- | -- | -- | 300 |
| Eau | 380 | 380 | 380 | 380 | 380 |
| pH de la suspension | 3,3 | 3,3 | 3,3 | 3,3 | 3,2 |
| % d'acide salicylique dans la suspension au moment de l'introduction ASA | 0,07 | 0,10 | 0,09 | 0,09 | 0,06 |

| Stabilité 1 mois dans le lyophilisat oral conservé à température ambiante 1 mois | 0,13 | 0,12 | 0,12 | 0,04 | 0,11 |
|---|---|---|---|---|---|
| Stabilité 13 mois dans le lyophilisat oral conservé à température ambiante environ 13 mois | 0,30 | 0,32 | 0,21 | 0,37 | 0,34 |

**Exemple 3 : Forme d'acide acétylsalicylique à 320 mg**

|  | 3.1 | 3.2 | 3.3 | 3.4 |
|---|---|---|---|---|
| Acide acétyl salicylique | 320 | 320 | 320 | 320 |
| Paracétamol enrobé (T = 89,6 %) | -- | -- | -- | 133,93 |
| Citrate de magnésium | 220 | 220 | 220 | 220 |
| Aspartam | 15 | 15 | 15 | 15 |
| Dextran 70 | 70 | 60 | 70 | 70 |
| Lactose | 225 | 235 | -- | 31,07 |
| Mannitol | -- | -- | 220 | -- |
| Eau | 400 | 400 | 400 | 400 |
| pH de la suspension | 3,3 | 3,3 | 3,3 | 3,3 |

| % d'acide salicylique dans la suspension au moment de l'introduction ASA | 0,15 | 0,12 | 0,15 | 0,12 |
|---|---|---|---|---|
| Stabilité 1 mois dans le lyophilisat oral conservé à température ambiante 1 mois | 0,08 | 0,07 | 0,07 | 0,07 |
| Stabilité 13 mois dans le lyophilisat oral conservé à température ambiante environ 13 mois | 0,16 | 0,18 | 0,16 | 0,39 |

| EXEMPLE 4 | | | |
|---|---|---|---|
| | 4.1 | 4.2 | 4.3 |
| Acétyl salicylate de lysine | 576 | 576 | 576 |
| Citrate de magnésium acide | 220 | 220 | 220 |
| Aspartam | 10 | 10 | 10 |
| Dextran 70 | 60 à 80 | -- | 60 à 80 |
| Polyvidone | -- | 50 à 70 | -- |
| Mannitol | 250 à 300 | -- | -- |
| Glycocolle | -- | 200 à 300 | 150 |
| Cellulose microcristalline | -- | -- | 100 à 150 |
| Eau | 600/700 | 500/600 | 500/600 |

| EXEMPLE 5 | | | |
|---|---|---|---|
| | **5.1** | **5.2** | **5.3** |
| Acétyl salicylate de lysine | 900 | 900 | 900 |
| Citrate de magnésium acide | 250 à 300 | 250 à 300 | 250 à 300 |
| Aspartam | 15 à 20 | 15 à 20 | 15 à 20 |
| Dextran 70 | 80 à 100 | -- | 80 à 100 |
| Polyvidone | -- | 70 à 100 | -- |
| Mannitol | 200 à 300 | -- | -- |
| Glycocolle | -- | 200 à 300 | 150 |
| Cellulose microcristalline | -- | -- | 100 |
| Eau | 700/800 | 600/700 | 600/700 |

| EXEMPLE 6 | | | |
|---|---|---|---|
| | **6.1** | **6.2** | **6.3** |
| Acide acétylsalicylique | 320 | 320 | 320 |
| Citrate de magnésium acide | 200 à 250 | 200 à 250 | 200 à 250 |
| Aspartam (ou autre édulcorant) | 10 à 15 | 10 à 15 | 10 à 15 |
| Dextran 70 | 60 à 80 | -- | 60 à 80 |
| Polyvidone | -- | 50 à 80 | -- |
| Glycocolle | 100 à 150 | 100 à 150 | 100 |
| Cellulose microcristalline | -- | -- | 50 à 100 |
| Eau | 400/500 | 400/500 | 400/500 |

## EXEMPLE 7

|  | 7.1 | 7.2 | 7.3 |
|---|---|---|---|
| Acide acétylsali-cylique | 320 | 320 | 500 |
| Citrate de magné-sium acide | 220 | 220 | 150 à 200 |
| Edulcorant (as-partam ou saccha-rine sodique) | 10 à 15 | 10 à 15 | 10 à 15 |
| Dextran 70 | 60 à 70 | 60 à 70 | 60 à 80 |
| Acide citrique | 50 | 50 | 50 |
| Lactose | 100 | -- | 20 à 100 |
| Cellulose micro-cristalline | -- | 50 à 100 | -- |
| Eau | 400/500 | 400/500 | 400/500 |

## EXEMPLE 7 (suite)

|  | 7.4 | 7.5 |
|---|---|---|
| Acide acétylsa-licylique | 500 | 500 |
| Citrate de ma-gnésium acide | 150 | 300 |
| Edulcorant (as-partam ou sac-charine sodique) | 10 à 15 | 10 à 15 |
| Dextran 70 | 60 à 80 | 60 à 100 |
| Acide citrique | 50 | 50 |
| Lactose | -- | 300 |
| Cellulose mi-crocristalline | 50 à 100 | -- |
| Eau | 400/500 | 600/700 |

| EXEMPLE 8 | | | |
|---|---|---|---|
| | **8.1** | **8.2** | **8.3** |
| Acide acétylsalicylique | 320 | 500 | 500 |
| Citrate de magnésium acide | 220 | 160 à 200 | 300 |
| Edulcorant (aspartam ou gesweet) | 10 à 15 | 10 à 15 | 10 à 15 |
| Dextran 70 | 60 à 70 | 60 à 70 | 60 à 100 |
| Phosphate disodique | 75 | 75 | 75 |
| Lactose | 70 à 100 | 20 à 100 | 250 à 300 |
| Eau | 400/500 | 400/500 | 600/700 |

EXEMPLE 9

| | 9.1 | 9.2 | 9.3 |
|---|---|---|---|
| Acide acétylsalicylique | 320 | 320 | 500 |
| Citrate de magnésium acide | 220 | 220 | 150 à 200 |
| Edulcorant | 10 à 15 | 10 à 15 | 10 à 15 |
| Dextran 70 | 60 à 70 | 60 à 70 | 60 à 70 |
| Hydroxyde d'aluminium | 130 | 130 | 130 |
| Lactose | 20 à 50 | -- | -- |
| Cellulose microcristalline | -- | 20 à 50 | 20 à 50 |
| Eau | 400/500 | -- | -- |

## EXEMPLE 9 (suite)

|  | 9.4 | 9.5 |
|---|---|---|
| Acide acétylsali-cylique | 500 | 500 |
| Citrate de magné-sium acide | 150 à 200 | 300 |
| Edulcorant | 10 à 15 | 10 à 15 |
| Dextran 70 | 60 à 70 | 60 à 100 |
| Hydroxyde d'alu-minium | 130 | 130 |
| Lactose | -- | 250 à 350 |
| Cellulose micro-cristalline | 20 à 50 | -- |
| Eau | -- | 600/700 |

| EXEMPLE 10 | | | |
|---|---|---|---|
|  | 10.1 | 10.2 | 10.3 |
| Acide acétylsalicylique microencapsulé à l'éthyl cellulose (titre = 95 %) | 337 | 526 | 526 |
| Citrate de magnésium acide | 220 | 150 à 200 | 250 à 300 |
| Edulcorant | 10 | 10 | 10 |
| Dextran 70 | 60 à 70 | 60 à 70 | 60 à 70 |
| Lactose | 100 à 150 | 50 à 100 | 250 à 350 |
| Eau | 400/500 | 400/500 | 700/800 |

| EXEMPLE 11a, 11b, 11c | | | |
|---|---|---|---|
| **EXEMPLES** | **11a** | **11b** | **11c** |
| Acide acétylsalicylique (ASA) | 320 | 320 | 320 |
| Citrate de magnésium acide | 220 | -- | -- |
| Citrate de magnésium neutre | -- | 230 | 230 |
| Aspartam | 15 | 15 | 15 |
| Dextran 70 | 70 | 70 | 70 |
| Lactose | 225 | 215 | 165 |
| Acide citrique | -- | -- | 50 |
| eau | 400 | 400 | 400 |
| Masse unitaire après lyophilisation | 850 | 850 | 850 |
| pH de la suspension avant lyophilisation | 3,3 | 4,2 | 3,3 |
| % d'acide salicylique (% par rapport à l'ASA) <u>dans la suspension</u> | 0,021 | 0,026 | 0,035 |
| <u>dans le lyophilisat oral</u> Après 7 jours (conservation en flacon de verre) | 0,025 | 0,080 | 0,037 |
| Après 15 jours sous blister PVDC/Al | 0,036 | 0,104 | 0,021 |

| EXEMPLE 12a, 12b, 12c | | | |
|---|---|---|---|
| EXEMPLES | 12a | 12b | 12c |
| Acide acétyl salicylique (ASA) | 320 | 320 | 320 |
| Citrate de magnésium acide | 220 | -- | -- |
| Citrate de magnésium neutre | -- | 230 | 230 |
| Aspartam | 15 | 15 | 15 |
| Dextran 70 | 70 | 70 | 70 |
| Lactose | 95 | 85 | -- |
| Hydroxyde d'aluminium | 130 | 130 | -- |
| Glycocolle | -- | -- | 215 |
| Eau | 400 | 400 | 400 |
| Masse unitaire après lyophilisation | 850 | 850 | 850 |
| pH de la suspension avant lyophilisation | 3,2 | 4,5 | 4,5 |
| % d'acide salicylique (% par rapport à l'ASA) dans la suspension | 0,030 | 0,026 | 0,038 |
| dans le lyophilisat oral après 11 jours (conservation en flacon de verre) | 0,029 | 0,222 | 0,166 |
| Après 12 jours sous blister PVDC/al | 0,025 | 0,244 | 0,186 |

| EXEMPLE 13a et 13b | | |
|---|---|---|
| EXEMPLES | 13a | 13b |
| Acide acétyl salicylique microencapsulé (T = 95 %) | 332 | 332 |
| Citrate de magnésium acide | 220 | -- |
| Citrate de magnésium neutre | -- | 230 |
| Aspartam | 15 | 15 |
| Dextran 70 | 70 | 70 |
| Lactose | 213 | 203 |
| Eau | 400 | 400 |
| Masse unitaire après lyophilisation | 850 | 850 |
| pH de la suspension avant lyophilisation | 3,5 | 4,4 |
| % d'acide salicylique (% par rapport à l'ASA) dans la suspension | 0,212 | 0,190 |
| dans le lyophilisat oral Après 7 jours (conservation en flacon de verre) | 0,152 | 0,272 |

EP 0 643 968 A1

## EXEMPLES COMPARATIFS A et B

On a préparé des formes moulées par lyophilisation à base d'acide acétylsalicylique contenant des excipients "classiques".

|  | A | B |
|---|---|---|
| Acide acétyl salicylique | 500 | 500 |
| Poloxamère 188 | 17 | 67 |
| Dextran 70 | 50 | 50 |
| Arginine | 483 | 483 |
| Glycocolle | 450 | 450 |
| Saccharinate sodique |  | 10 |
| Chlorure de sodium |  | 25 |
| Eau | 1550 | 1500 |

### Procédé de fabrication :

On dissout le dextran 70 et le poloxamère 188 dans l'eau. On incorpore le glycocolle et l'arginine de façon homogène. On refroidit sous agitation la suspension obtenue jusqu'à une température d'environ -5/-7° C. On ajoute l'acide acétyl salicylique tout en continuant de mélanger et en maintenant le mélange à -5° C.
On répartit en alvéoles thermoformés.
On congèle à -40°/-45° C.
On lyophilise.

### Analyse des lyophilisats obtenus :

Après 10 jours de conservation à température ambiante :

|  | A | B |
|---|---|---|
| Teneur en acide acétyl salicylique | 36,5 % | 59,35 % |
| Teneur en acide salicylique | 63,5 % | 40,65 %. |

## Revendications

1. Forme pharmaceutique moulée pour administration orale, stable, à base d'acide acétyl salicylique, caractérisée en ce qu'elle comprend un mélange d'un composé a) choisi parmi l'acide acétyl salicylique et ses sels pharmaceutiquement acceptables et un citrate b) choisi parmi le citrate de magnésium neutre et le citrate de magnésium acide, ce mélange étant finement réparti dans une matrice poreuse à base d'un liant pharmaceutiquement acceptable.

2. Forme pharmaceutique selon la revendication 1, dans laquelle le citrate est le citrate de magnésium acide.

3. Forme pharmaceutique selon la revendication 1, dans laquelle le citrate est présent en un rapport molaire de 0,1 à 1 par rapport à l'acide acétyl salicylique ou ses sels.

4. Procédé de préparation d'une forme pharmaceutique selon la revendication 1 comprenant le mélange à une température inférieure ou égale à la température ambiante, dans un milieu aqueux,
   - d'un composé choisi parmi l'acide acétylsalicylique et ses sels pharmaceutiquement acceptables,

14

- d'un citrate choisi parmi le citrate de magnésium neutre et le citrate de magnésium acide,
- d'un liant,

pour former une suspension homogène, la répartition de la suspension dans des alvéoles, puis la lyophilisation de la suspension.

5. Procédé selon la revendication 4, dans lequel on mélange le composé choisi parmi l'acide acétylsalicylique et ses sels pharmaceutiquement acceptables avec le citrate de magnésium à sec puis on incorpore à ce mélange une solution aqueuse du liant, à température ambiante.

6. Procédé selon l'une des revendications 4 ou 5, caractérisé en ce que la suspension a un pH inférieur ou égal à 4,5.

Office européen
des brevets

**RAPPORT DE RECHERCHE EUROPEENNE**

Numero de la demande

EP 94 40 2027

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| D,A | FR-A-2 670 675 (LABORATOIRE L. LAFON)<br>* le document en entier *<br>----- | 1-6 | A61K31/60<br>A61K9/20 |
| | | | **DOMAINES TECHNIQUES RECHERCHES (Int.Cl.6)** |
| | | | A61K |

Le présent rapport a été établi pour toutes les revendications

| Lieu de la recherche | Date d'achèvement de la recherche | Examinateur |
|---|---|---|
| LA HAYE | 5 Décembre 1994 | VENTURA AMAT, A |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)